# EUROPEAN PATENT APPLICATION

(11) **EP 3 175 705 A1**
(43) Date of publication of application: **07.06.2017**
(21) Application number: 15197903.6
(22) Date of filing: 03.12.2015
(51) Int. Cl.: A01K 67/00, C12Q 1/68, C12N 15/113, C12N 15/85

(54) **METHOD FOR PRODUCING ARTIFICIAL PIRNAS AND USES THEREOF FOR SILENCING GENE EXPRESSION**

(71) Applicant: European Molecular Biology Laboratory, 69117 Heidelberg (DE)
(72) Inventor: Pillai, Ramesh, 38120 St. Egreve (FR); Homolka, David, 38120 Saint-Egréve (FR); Pandey, Radha Raman, 38100 Grenoble (FR)
(74) Representative: Krauss, Jan

(57) **Abstract**

The present invention relates to a method for producing at least one artificial piRNA, comprising determining an endogenous piRNA population in a cell of an organism of interest; selecting a sub-population of said endogenous piRNA population based on binding of a MILI protein and/or an equivalent PIWI protein to said sub-population; identifying the sequence of at least one piRNA as selected, attaching said at least one piRNA sequence as identified 5' to the sequence of the transcript of a gene of interest, in order to generate at least one piRNA template, and slicing said piRNA template through binding of a MILI protein to said template and subsequent slicing of said bound piRNA template following a binding of MILI protein and/or MIWI2 protein in order to generate at least one artificial piRNA. The method can be used to produce pharmaceutical compositions comprising at least one artificial piRNA as produced according to the invention. Other aspects relates to a method for silencing the expression of a gene of interest in an organism of interest, comprising administering to said organism an effective amount of a pharmaceutical composition according to the invention, and a method for produing a non-genetically modified organism having an artificially silenced expression of at least one gene of interest based on the method of the invention.

## Description

The present invention relates to a method for producing at least one artificial piRNA, comprising determining an endogenous piRNA population in a cell of an organism of interest; selecting a sub-population of said endogenous piRNA population based on binding of a MILI protein and/or an equivalent PIWI protein to said sub-population; identifying the sequence of at least one piRNA as selected, attaching said at least one piRNA sequence as identified 5' to the sequence of the transcript of a gene of interest, in order to generate at least one piRNA template, and slicing said piRNA template through binding of a MILI protein to said template and subsequent slicing of said bound piRNA template following a binding of MILI protein and/or MIWI2 protein in order to generate at least one artificial piRNA. The method can be used to produce pharmaceutical compositions comprising at least one artificial piRNA as produced according to the invention. Other aspects relates to a method for silencing the expression of a gene of interest in an organism of interest, comprising administering to said organism an effective amount of a pharmaceutical composition according to the invention, and a method for produing a non-genetically modified organism having an artificially silenced expression of at least one gene of interest based on the method of the invention.

### Background of the invention

Animal gonads express a set of 24-30 nucleotide (nt) small RNAs called PIWI-interacting RNAs (piRNAs) that together with their partner PIWI protein constitute a transposon defense system (Houwing et al., 2007). Sequence complementarity to host transposable elements allows piRNAs to guide associated PIWI proteins to target nucleic acids. Cytoplasmic PIWI proteins are small RNA guided nucleases (slicers) that guide endonucleolytic cleavage of transposon targets, while nuclear PIWI proteins assemble silencing complexes on target genomic loci to mediate transcriptional silencing (Ghildiyal and Zamore, 2009).

The majority of piRNAs originate from RNA polymerase II transcription units called piRNA clusters. These are transcribed into 50-100 kilobases (kb) long, capped and polyadenylated precursor transcripts (Li et al., 2013). Precursors are then exported to the cytoplasm for processing as most known piRNA biogenesis factors are cytoplasmic proteins, and are resident in perinuclear granules called nuage. The single-stranded cluster transcripts are then converted into tens of thousands of piRNAs via a process termed primary processing (Brennecke et al., 2007).

*Drosophila* piRNA precursors were shown to contain non-sequence conserved cis-acting RNA elements (Ishizu et al., 2015) or piRNA trigger sequences (PTS) (Homolka et al., 2015), that when fused to any heterologous transcript can direct them into primary processing. These elements provide binding sites for piRNA biogenesis factors (Ishizu et al., 2015), and act in a directional (5'→3') manner by converting any sequence downstream into thousands of primary piRNAs with a prominent bias for carrying a uridine at the 5' end (1U). Although primary biogenesis pathway and factors involved seem to be conserved, it is still unclear whether the use of such signals to initiate processing is a common feature across the animal kingdom.

Transposon silencing in the mouse embryonic male germline is secured by the slicer-competent cytoplasmic MILI (Di Giacomo et al., 2013) and the slicer-inactive nuclear MIWI2 that is proposed to recruit transcriptional silencing complexes on genomic transposon loci (De Fazio et al., 2011). It is known that MILI (Aravin et al., 2008; Kuramochi-Miyagawa et al., 2008) and its slicer activity (De Fazio et al., 2011) is critical for biogenesis of most of the piRNAs associating with MIWI2, but the molecular mechanisms involved in this process are currently not known.

US 2014-0275216 relates to compositions and methods for the alteration of neuronal methylation by synthetic piRNAs or by alteration of piRNA function. Such alterations allegedly find use in the regulation and control of neural gene expression and concomitant neural functions. Further provided are systems and methods for the identification of target sites for regulation by piRNAs. A synthetic piRNA molecule can comprise a chemical modification or a nucleotide with at least one chemical modification. Also antisense oligonucleotides are proposed having a sequence complementary to an endogenous piRNA found in neural tissue. Lastly, a method for altering neural gene expression comprising: administering a synthetic piRNA to a subject under conditions such that methylation of a regulatory sequence regulating said gene is altered. The methods as proposed in US 2014-0275216 differ from the present application.

US 2009-0062228 discloses a method for regulating the expression of a target gene in a cell, comprising introducing into the cell a small single stranded RNA or analog thereof (piRNA) that: (i) selectively binds to proteins of the Piwi or Aubergine subclasses of Argonaute proteins relative to the Ago3 subclass of Argonaute proteins, (ii) forms an RNP complex (piRC) with the Piwi or Aubergine proteins, and, (iii) induces transcriptional and/or post-transcriptional gene silencing, wherein the piRNA induces transcriptional and/or post-transcriptional gene silencing of the target gene. Also, a polynucleotide is disclosed comprising two or more concatenated piRNAs, each of said piRNAs comprise a small single stranded RNA or analog thereof that: (i) selectively binds to proteins of the Piwi or Aubergine subclasses of Argonaute proteins relative to the Ago3 subclass of Argonaute proteins, (ii) forms an RNP complex (piRC) with the Piwi or Aubergine proteins, and, (iii) induces transcriptional and/or post-transcriptional gene silencing. The piRNAs can be of the same or different sequences.

In view of the above, it is an object of the present invention to provide new tools and methods in order to enable further beneficial uses in the field of piRNAs, in particular with respect to transcriptional control of targeted genes. Other aspects and objects will become apparent for the person of skill upon reading the following more detailed description of the invention.

According to a first aspect thereof, the above objects have been solved by the present invention by providing a method for producing at least one artificial piRNA, comprising the steps of: a) determining an endogenous piRNA population in a cell of an organism of interest; wherein said organism of interest is capable of methylating its DNA and/or histones; b) selecting a sub-population of said endogenous piRNA population based on binding of a MILI protein and/or an equivalent PIWI protein to said sub-population; c) optionally, identifying the sequence of at least one piRNA as selected in step b), d) fusing/attaching said at least one piRNA sequence as identified to the antisense sequence of a transcript of a gene of interest, in order to generate a piRNA template, and e) slicing said piRNA template through binding of a MILI protein to said template and subsequent slicing of said bound piRNA template following a binding of MILI protein and/or MIWI2 protein and/or an equivalent PIWI protein that has small RNA-guided endoribonuclease (slicer) activity, in order to generate at least one artificial piRNA. The method optionally and preferably involves the step of isolating said at least one artificial piRNA from said cell of said organism of interest.

Here, the inventors as an embodiment designed a strategy to create artificial piRNAs in the mouse germline. Such artificial sequences can be created to any target sequence of interest. The created small RNAs in the mouse associate with endogenous proteins called MILI and MIWI2. Artificial piRNAs shall be understood as not naturally occurrring in the cell of the organism of interest as mentioned above (e.g. in a germline cell but in a somatic cell, and/or in the organism of interest as such (e.g. not in the mouse as used, but e.g. in a human).

The at least one piRNA sequence as identified can be fused or attached (or can be positioned) in the 5' and/or 3'-position (i.e. upstream and/or downstream) to the antisense sequence of a transcript of a gene of interest in order to generate the piRNA template. Preferred is the 5'-attachment (i.e. as the "head") to the antisense sequence of a transcript of a gene of interest.

In the context of the present invention, the terms "MILI (PIWIL2)" and "MIWI2 (PIWIL4)" shall also include the piwi-like genes of organisms of interest other than the mouse, wherein the PIWI proteins bind to piRNAs, can have a small RNA-guided endoribonuclease (slicer) activity, and wherein said PIWI protein preferably is a nuclear protein (such as MIWI2), and wherein said organism of interest is capable of methylating its DNA and/or histones, and preferably is capable of CpG methylation. "Equivalent PIWI protein that has small RNA-guided endoribonuclease (slicer) activity similar to MILI" shall mean that PIWI proteins are included stemming from organisms of interest other than the mouse that also exhibit or confer a slicer activity that is preferably essentially the same, but at least functionally similar to the MILI activities as described herein, and in the respective literature as cited. Preferably, the equivalent PIWI proteins are homologous in their amino acid sequence to either MILI or MIWI2, that is, they are identical, to at least 99%, at least 95%, at least 90%, and at least 80% identical on the amino acid level, "small RNA-guided" shall mean that the activity of the enzyme depends on the binding to RNA-fragments as described herein (see, e.g., figures). Preferably, the size of the fragments of RNA as bound is below 60 nucleotides, preferably below 50 nucleotides, more preferred below 40 nucleotides. Most preferred are fragments having the size of piRNA-fragments or their precursors (see figures).

*D. melanogaster*, for example, only shows very low levels of adenine methylation during the earliest stages of Drosophila embryogenesis.

The methods of the present invention can be performed in vivo or in vitro, in particular in laboratory animals, or in culture.

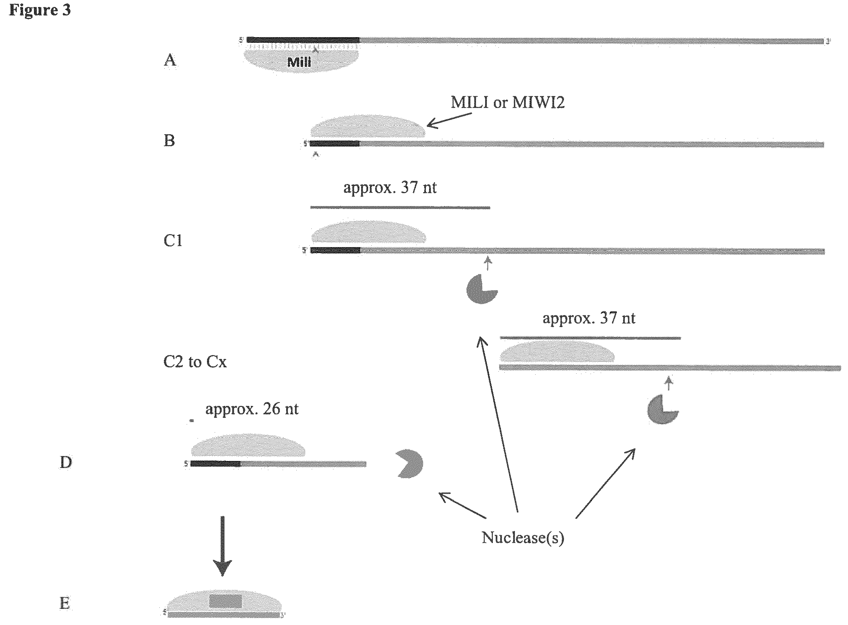

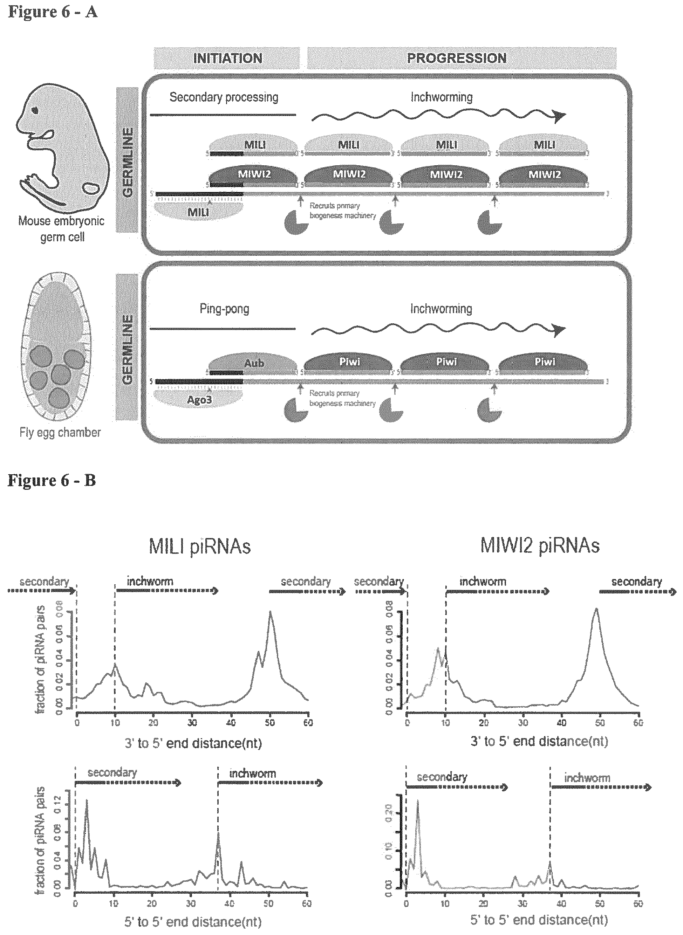

In a preferred method of the invention, for example in the mouse or rat context, an endogeneous piRNA population in a germ cell of the mouse or rat is determined by selecting a sub-population of said endogenous piRNA population through binding of a MILI protein to said sub-population, wherein part or all of the endogenous piRNA population is provided as a reporter construct in the mouse or rat that is subsequently bound by the MILI protein and cut, following a binding of the MILI protein (in the cytoplasm) and/or MIWI2 protein (in the nucleus). The bound and cut sequences are then selected, for example based on the abundancy (amount) of the piRNAs as cut. Then, the sequence(s) of at least one piRNA as selected is identified, and the piRNA is fused 5' to the sequence of the transcript of a gene of interest, in order to generate a piRNA template. In this context, the piRNA(s) as identified function like a "locomotive" at the head of the RNA of interest. The 5' fusion/attachment then causes slicing of the piRNA template through binding of a MILI protein to said template, and subsequent slicing of said bound piRNA template following a binding of MILI protein and/or MIWI2 protein in order to generate at least one artificial piRNA. In a longer piRNA-template, several piRNAs are successively generated (see Figures 6 and 3).

The invention also includes "hybrid" piRNA templates, i.e. the endogenous piRNA as identified can be fused to a target nucleic acid from a different cell or organism, e.g. a germline cell piRNA but a somatic cell target, and/or a mouse piRNA and a human target.

The method accordig to the invnetion thus allows creation of artificial small RNAs that can target any genomic locus in the (e.g.) mouse genome for epigenetic silencing by recruitment of DNA methylation or histone modifications. This allows manipulation of the gene expression without changing the underlying DNA sequence (non-genetically modified organisms).

The artificial small RNA can guide the MILI endonuclease to destroy target RNAs in the cytoplasm, or guide nuclear MIWI2 to target genomic loci for transcriptional repression by recruiting DNA methylation and/or histone modification. Specificity of the process is ensured by sequence complementarity between small RNA and the target nucleic acid.

Here the inventors surprisingly demonstrated that MILI slicing licenses a target transcript to enter the piRNA biogenesis pathway to generate a series of 1U-containing piRNAs in the 5'→3' direction, a process previously termed in flies as inchworming (Homolka et al., 2015). Such piRNAs associate with both MILI and MIWI2 (Figure 6). However, since MIWI2 piRNA levels are disproportionately affected in the MILI-slicer mutant (De Fazio et al., 2011), it is believed that this process primarily serves to populate MIWI2 with piRNAs. Inchworming represents a very efficient mechanism by which germ cells can extract a large amount of sequence information in the form of small RNAs from the targets of PIWI slicing.

This is a similar mechanism that links cytoplasmic PIWI slicing to nuclear piRNA biogenesis as in operation in the fly ovarian germline (Han et al., 2015; Homolka et al., 2015; Mohn et al., 2015). Pointing to a mechanistic similarity, orthologous factors are involved in loading the nuclear PIWI protein in the fly and mouse systems (Figure 6A). However, despite being conceptually related, the fly and mouse inchworming processes differ significantly. First, in the 3-PIWI system of the fly ovarian germline, cytosolic Ago3 slices the target to load the secondary piRNA into Aubergine, while all downstream inchworm piRNAs are primarily reserved for nuclear Piwi (Figure 6B). In contrast, the 2-PIWI system of the mouse testicular germline uses the generated secondary and inchworm piRNAs to load both MILI and MIWI2 with no apparent specificity. Second, the spacing between consecutive piRNAs is longer in mice (-37 nt between 5' ends), requiring extensive trimming to mature the 3' ends.

Since both MILI and MIWI2 are recipients of the inchworming process, the same piRNAs can be used by MILI to silence transposon transcripts in the cytoplasm, while MIWI2 will repress transposon genomic loci in the nucleus. Preferred are methods of the invention, wherein said slicing is performed in the nucleus using MIWI2 protein.

Preferred is a method according to the invention, wherein said cell that is used to determine an endogenous piRNA population and/or the other steps of the present method, is selected from an animal or plant cell or cell line, such as, for example, a germ line cell, in particular a female cell, a stem cell, such as, for example, an embryonic stem cell, a reprogrammed stem cell, a somatic cell, an ovarian somatic cell, a tissue, and recombinant cells or tissues thereof. Preferably, human embryonic stem cells and methods that necessarily involve the destruction of the human embryos are excluded.

The method according to the invention involves DNA and/or histone methylation, thus, the organism of interest is capable of methylating its DNA and/or histones, and preferably is selected from an animal, a mammal, such as, for example, a mouse, rat, cat, dog, horse, goat, sheep, monkey, or human, or a plant, such as, for example, a crop plant, and recombinant animals or plants thereof. Since PIWI-proteins are widely conserved, the method can be applied both to plants and animals.

The nucleic acids as used in the context of the present invention can be enzymatically and/or synthetically (chemically) produced (e.g. using a synthesizer), as known to the person of skill and described in the art.

Preferred is the method according to the invention, wherein selecting the sub-population in step b) as above comprises identifying the population of endogenous piRNAs using a suitable method, such as, for example, deep sequencing, and/or immune-precipitation of MILI-bound endogeneous piRNAs, e.g. using MILI (or MILI-homolog for homologous proteins) specific antibodies.. This step serves as the basis in order to identify the starting "endogenous" population of piRNAs. Preferred are piRNAs having a perfect and/or highly functional binding motif for the MILI or the MIWI2 protein (or the relevant PIWI homolog in the organism of interest).

Preferably, selecting the sub-population can be based, at least in part, on the abundancy of the piRNA sequences as identified in the MILI-bound population in said cell. That is, the piRNAs with the highest frequencies are chosen for the further production as these piRNAs are deemed most relevant for the cell of the organism. Also, different templates can be selected as further described below. Preferred is a method according to the invention, wherein the number of different artificial piRNAs templates or artificial piRNAs as selected and produced is between 1 and 50, and preferably between 5 and 15.

The piRNA sequences as identified and selected are then preferably cloned into a reporter construct, such as, for example, the *Rosa26* promoter-driven DsRed reporter construct and cassette as disclosed herein. Preferred is the method according to the invention, wherein selecting the sub-population in step b) of the method comprises using a reporter construct comprising between 10 to 50 complementary sites for MILI-bound piRNAs. Other embodiments have 15, 20, 25, 30, 35, 40 or 45 complementary sites for MILI-bound piRNAs. The number of piRNAs as identified may depend from the organism and/or the type of cell that forms the source of the piRNAs.

In another aspect of the invention, the proteins/genes as required for piRNA generation (see, for example, Figure 3) can be provided as recombinant proteins into a cell that otherwise lacks certain components as required (e.g. a somatic cell). Thus, further preferred is a method according to the invention, wherein the proteins as used, for example MILI and MIWI2, are at least in part recombinantly introduced into the cell and expressed. Optimally, the cell is a cell of an organism of interest. Other genes/proteins may include nucleases (e.g. for trimming), and/or additional proteins that are required, and may bind and stabilize a complex that produces piRNAs. Techniques as required for cloning, transfection and expression of recombinant genes are well known to the person of skill.

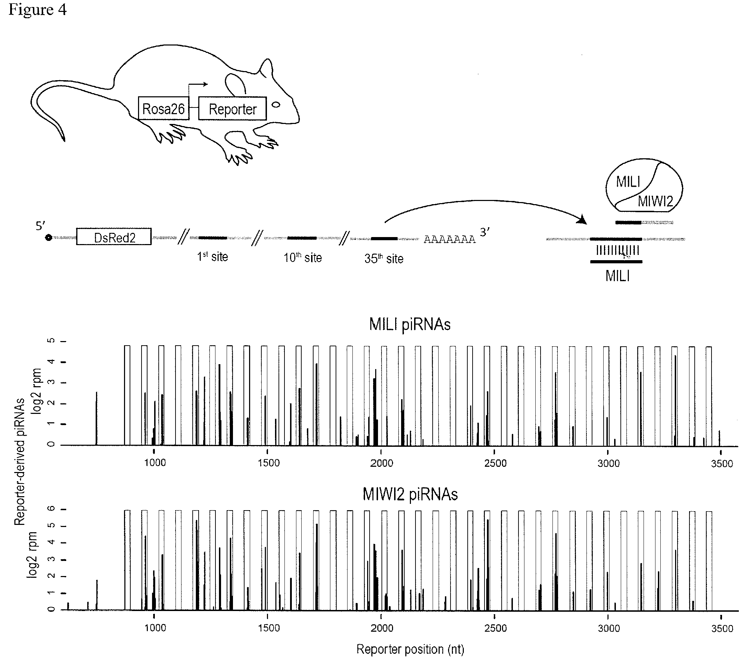

Also in the reporter-embodiment as described, once the population of endogenous piRNAs has been established using the reporter construct (see, for example, Figure 4), a sub-population of said endogenous piRNAs as cloned in the reporter is selected. Preferably, selecting the sub-population is based, at least in part, on the abundancy of the piRNA sequences in said cell. That is, the piRNAs with the highest frequencies are chosen for the further production as these piRNAs are deemed most relevant for the cell of the organism.

Once the piRNAs are selected, their sequence(s) of at least one piRNA can be identified. Respective methods are well known to the person of skill.

Then, once the sub-population of piRNAs is selected, the piRNA(s) is/are attached 5' to a nucleic acid sequence of the gene of interest, in order to generate at least one piRNA template. The sequence is preferably based on the transcript of the gene of interest, according to the either partly or fully ripened/processed mRNA. Also, cDNA sequences may be used.

The choice of the gene of interest depends on the intended use of the piRNA molecules as used, either in therapy, research and/or diagnosis. Preferred is the method according to the invention, wherein said gene of interest is selected from genes involved/causing in cancer, neurological diseases, immunological diseases, allergy, metabolic diseases, fertility (e.g. reproductive rate or number), animal production (e.g. amount of meat or milk), genes essential for cell growth and/or development, for mRNA degradation, for translational repression, and/or for transcriptional gene silencing.

The invention for the first time allows to produce artificial piRNAs from virtually any target (desired) gene of interest, independent from piRNA clusters.

As mentioned above, optionally, the at least one artificial piRNA as produced can be isolated from said cell of said organism of interest. The piRNAs can be labelled (e.g. radioactively) and/or attached, admixed, encapsulated, conjugated or otherwise associated with liposomes, polymers, receptor targeted molecules, antibodies or parts thereof, or drug moieties.

Further preferred is a method according to the invention, further comprising identifying the at least one artificial piRNA as produced. In this step, the population of the piRNAs as produced from the template is identified (e.g. by sequencing). The population can also be further selected, in order to isolate specific piRNAs that prove to be, for example, particularly effective or is/are desirable for further applications (e.g. therapy).

Another aspect of the invention then relates to a pharmaceutical composition comprising at least one artificial piRNA as produced according to a method according to the invention, together with pharmaceutically acceptable salts, esters or salts of such esters, or bioequivalent compounds thereof, admixed, encapsulated, conjugated or otherwise associated with liposomes, polymers, receptor targeted molecules, oral, rectal, topical or other formulations that assist uptake, distribution and/or absorption, and optionally further comprising penetration enhancers, carrier compounds, and/or transfection agents.

Another aspect of the invention then relates to the pharmaceutical composition as above for use in medicine. Another aspect of the invention then relates to the pharmaceutical composition as above for use in the treatment or prevention of diseases caused (directly or indirectly) by the gene of interest, such as, for example, cancer, neurological diseases, immunological diseases, allergy, metabolic diseases. The therapy involves transcriptional silencing of said gene by DNA methylation or histone methylation.

Another aspect of the invention then relates to a method for silencing the expression of a gene of interest (as above) in an organism of interest that is capable of methylating its DNA and/or histones, comprising administering to said organism an effective amount of the pharmaceutical composition according to the invention. This aspect involves both medical and cosmetic applications, as well as can be used in the breeding of non-human animals or plants. Nevertheless, preferred is a method according to the invention, wherein said organism suffers or is likely to suffer from a condition or disease that can be treated and/or ameliorated by silencing the expression thereof.

Another aspect of the invention then relates to the use of the pharmaceutical composition according to the invention for silencing the expression of a gene of interest (as above) in an organism of interest that is capable of methylating its DNA and/or histones. This aspect involves both medical and cosmetic applications, as well as can be used in the breeding of non-human animals or plants.

In the present invention, the inventors have shown that they can create artificial small RNAs at will in the mouse germline (producer line). The generated artificial small RNAs function by cleaving target RNAs in the cytoplasm or transcriptionally repress target genomic loci by recruitment of DNA methylation/histone modifications. Mice carrying genomic targets for the artificial small RNAs are created (sensor line). After crossing of the producer and sensor mouse lines, it is checked whether the targets are silenced by the artificial small RNAs.

Another aspect of the invention then relates to a method for produing a non-genetically modified organism having an artificially silenced expression of at least one gene of interest, comprising the steps of: a) determining an endogenous piRNA population in a germ line or stem cell of said organism; wherein said organism is capable of methylating its DNA and/or histones; b) selecting a sub-population of said endogenous piRNA population based on binding of a MILI protein and/or an equivalent PIWI protein to said sub-population, c) optionally, identifying the sequence of at least one piRNA as selected in step b), d) fusing/attaching said at least one piRNA sequence as identified to an antisense sequence of a gene of interest, in order to generate at least one piRNA template, and e) introducing said piRNA template into the germ line or stem cell of said organism, and f) generating said non-genetically modified organism through breeding and removing of said piRNA template, e.g. by crossing. This aspect of the invention makes use of the fact that methylation of DNA and/or histones can be inhereted and thus influences gene expression without that the underlying DNA sequence is modified and/or transgenic genes or constructs remain in the genome of the modified organism. The organism is capable of methylating its DNA and/or histones, and preferably is selected from an animal, a mammal, such as, for example, a mouse, rat, cat, dog, horse, goat, sheep, monkey, or human, and respective recombinant animals. Since PIWI-proteins are widely conserved, the method can be applied to all animals.

Another aspect of the invention then relates to a non-genetically modified organism having an artificially silenced expression of at least one gene of interest that is produced/producible according to a method according to the invention. The organism is capable of methylating its DNA and/or histones, and preferably is selected from an animal, a mammal, such as, for example, a mouse, rat, cat, dog, horse, goat, sheep, monkey, or human, or a plant, such as, for example recombinant animals thereof.

The present invention will now be illustrated further in the following non-limiting examples, with reference to the accompanying figures. For the purposes of the invention, all publications, patent applications, patents, and other references mentioned herein are incorporated by reference in their entirety.

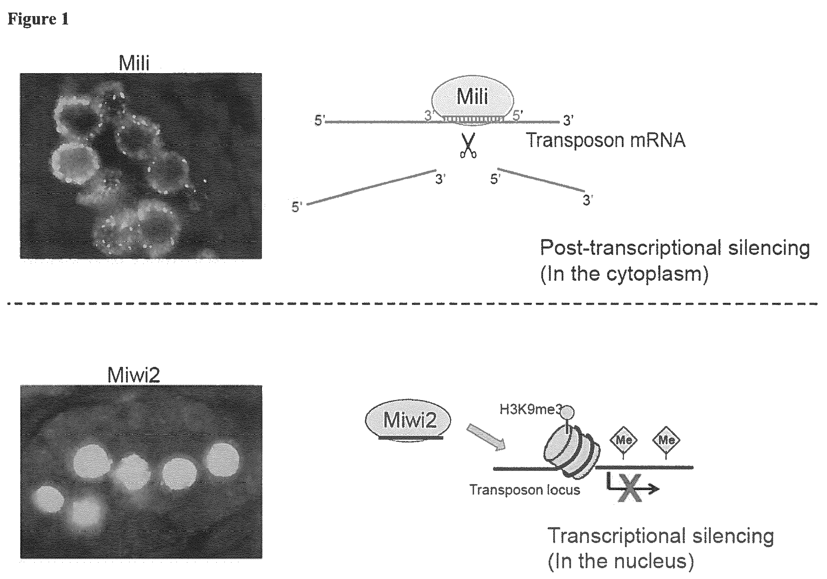

Figure 1 shows a schematic overview of the transposon silencing mechanisms in the mouse cytoplasm involving Mili-siRNA complex (top half) and nuclease activity, and the nucleus involving Miwi2-siRNA complex (bottom half) and epigenetic silencing based on DNA methylation and/or histone methylation.

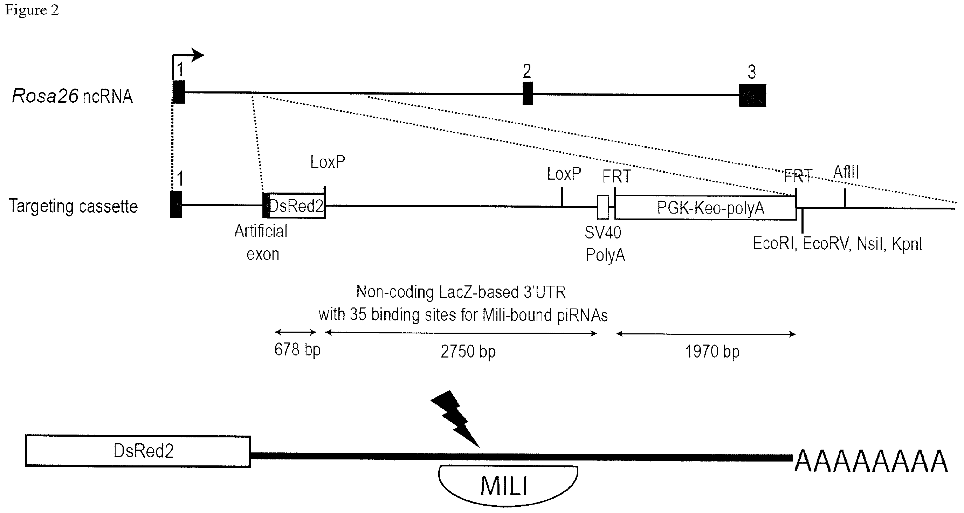

Figure 2 shows the structure of a *Rosa26* promoter-driven DsRed reporter construct and cassette (middle). This embodiment/construct has 50 binding sites for Mili-bound piRNAs.

Figure 3 shows a schematic overview of part of the method according to the present invention. A) attachment/binding of Mili to a piRNA (dark region)/target RNA fusion/template (light gray region) and cleavage/slicing of the strand/fusion (small triangle). B) Subsequent binding of Mili or Miwi2 protein to the sliced, and C1) slicing of approximately 37 nt. C2 to Cx) repetitive slicing (e.g. by "inchworming") of approximately 37 nt pre-piRNAs. D) Trimming of the approximately 37 nt pre-piRNA to approximately 26 nt piRNA, bound to Mili or Miwi2 protein, and E) final Mili or Miwi2 protein-piRNA complex.

Figure 4 shows that MILI slicing of a target triggers production of MIWI2 piRNAs in a 5'→3' direction. (A) The ubiquitous *Rosa26* promoter-driven DsRed reporter transcript has a 3'-UTR with (in this case) 35 perfectly complementary binding sites (dark regions) for different MILI-bound piRNAs. Slicer cleavage of the transcript by MILI leads to accumulation of reporter-derived piRNAs in both MILI and MIWI2. The 5' ends of such piRNAs were mapped along the transcript and those exceeding the abundance of 1 rpm were plotted. The abundance is shown in logarithmic scale.

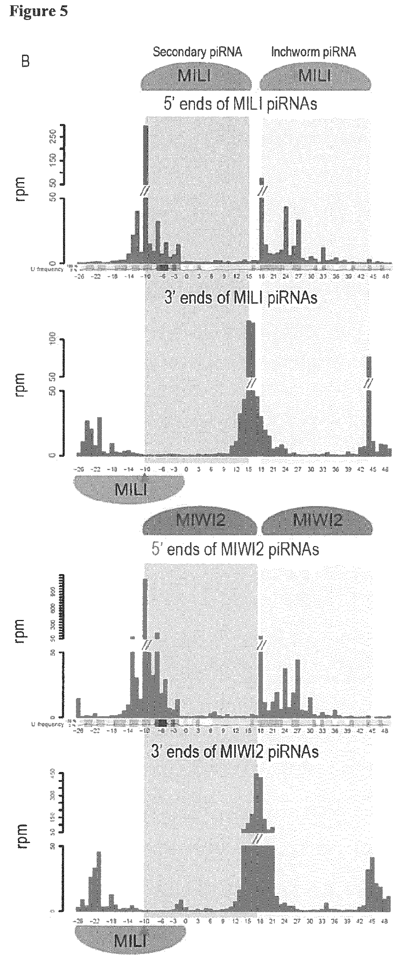

Figure 5 shows mapping of the 5' and 3' ends of reporter-derived artificial piRNAs relative to the 5' end (nucleotide position -1) of the targeting MILI piRNA. Read counts were aggregated for the 35 individual MILI-targeted sites. The y-axis was scaled to allow inspection of even low abundant reads. The 5' ends of secondary piRNAs (at position -10) are the most abundant, and are generated by MILI slicing. Another distinct set of 5' ends (inchworm piRNAs) map downstream from the 3' end of these secondary piRNAs (~at positions +18 to +33).

Figure 6 shows a schematic overview of MIWI2 piRNA biogenesis by inchworming and factors involved. A) Cytosolic PIWI slicing by MILI leads to loading of fragments from the target as new piRNAs in a nuclear PIWI (MIWI2) or cytosolic PIWI proteins (MILI). In flies, where three PIWI proteins are involved, there is a strict partitioning of secondary and inchworm piRNAs into distinct PIWI proteins. In contrast, in the mouse embryonic male germline, these piRNAs enter both MILI and MIWI2 without any apparent bias. B) Calculation of 3'-5' distances between the piRNAs produced from the DsRed region. A regular spacing with frequent -10-20 nt distance is noted.

### EXAMPLES

The repetitive nature of piRNA populations present in the mouse embryonic testes prevents an unambiguous assignment of endogenous piRNAs associating with MILI or MIWI2. To overcome this, the inventors created a mouse knock-in line expressing a reporter transcript that is a target for MILI slicing and tracked piRNA biogenesis initiated by MILI slicer activity in vivo. The inventors demonstrated that MILI slicing triggers the generation of non-overlapping 1U-containing piRNAs in a 5'→3' direction, and these are loaded into both MILI and MIWI2.

The inventors created a mouse knock-in line (hereafter referred to as *Rosa26-piKI*) that expresses a reporter targeted by multiple MILI-bound piRNAs. They then prepared small RNA libraries of MILI and MIWI2 complexes isolated from testes of new-born pups (post-natal day 0; P0) of the *Rosa26-pi+*/*KI* genotype. Analysis indicates presence of reporter-derived reads in both MILI and MIWI2 libraries, with many originating from within the region predicted to be cleaved by MILI. PIWI proteins slice a target RNA at a fixed distance (10 nt) downstream from the 5' end of the piRNA guide (De Fazio et al., 2011; Reuter et al., 2011). Indeed, majority of the reporter-derived piRNAs have their 5' ends at a distance (position -10) consistent with generation by MILI slicing (Figure 6B). Additionally, since the targeting MILI piRNAs that the inventors chose in the reporter design have a uridine as the first residue (1U), all the reporter-derived reads have an adenosine (A10) at the 10th position. By definition (Brennecke et al., 2007), these MILI- and MIWI2-loaded sequences are secondary piRNAs as a PIWI protein generates their 5' ends.

A putative nuclease generating the 5' ends cleaves the reporter -37 nt downstream of the 5' end of the secondary piRNA, which is the most frequent 5' to 5' distance between the piRNAs. Subsequently, the 3' end of the secondary piRNA is matured by trimming to obtain -26 nt MILI or -28 nt MIWI piRNAs. This corresponds to -10 nt trimming, which is seen as the most frequent 3' to 5' distance between the piRNAs (Figure 6B). The mouse reporter reveals how MILI slicing recruits the target transcript as a substrate for piRNA biogenesis. The downstream cleavage fragment is used to generate a series of non-overlapping piRNAs.

### Mouse strains

Mouse knock-in line (*Rosa26-pi*) expressing a DsRed reporter from the ubiquitous *Rosa26* locus was prepared. The reporter has a 3'-UTR containing perfectly complementary binding sites for 35 different MILI-bound piRNAs present in perinatal mouse testes.

### Small RNA Libraries

Mouse PIWI complexes were isolated by immunoprecipitation from testes of neonates (P0) and libraries of associated piRNAs were prepared for deep sequencing. When indicated, total small RNAs (19-40 nt) were used for library preparation.

### Generation of Rosa26-pi reporter knock-in mouse

For driving ubiquitous expression in the mouse system, we targeted exon 1 of the *Rosa26* locus in the mouse embryonic stem cell (ES) A9 line with a cassette carrying a piRNA reporter. The reporter contains the DsRed2 fluorescent protein coding sequence followed by a long 3' UTR based on the LacZ backbone (rendered noncoding by removing all ATGs) where we placed perfectly complementary sites for (in this case) 35 independent MILI-bound piRNAs. The piRNAs the inventors chose are those expressed abundantly in the embryonic/perinatal mouse male germline where PIWI proteins MILI and MIWI2 are co-expressed. The expectation is that the reporter would be expressed everywhere in the mouse, but silenced only in the embryonic/new-born testes (where complementary piRNAs are produced). The targeting cassette had the following elements: First, the dsRed coding sequence (Kozak-ATG-dsRed-STOP). Second, immediately downstream of the dsRed reporter we placed perfectly complementary binding sites for 35 independent piRNAs abundantly present in MILI complexes isolated from embryonic/new-born testes. Each piRNA binding site was separated from the neighboring ones by ~50 nt and the overall length of this region was 2750 bp. Third, flanking the region with complementarity to piRNAs, the inventors placed LoxP sites to enable its eventual removal by the Cre recombinase. Fourth, downstream of the last LoxP, the inventors inserted the SV40 polyA signal (131 bp) from pcDNA3.1. Fifth, downstream of the polyA signal, the inventors inserted the FRT-flanked Keo selection marker for screening targeted ES cells. Sixth, additional restriction enzymes for Southern screening were introduced further downstream (EcoRI, EcoRV, KpnI, NsiI).

The sequences encoding the following: DsRed2-LoxP-piRNAsites-in-LacZ-LoxP-SV40pA, were chemically synthesized (GeneArt) and inserted into the NurI-homology regions of a targeting construct for the *Rosa26* locus. The FRT-flanked Keo reporter was cloned in later to complete the targeting construct. Given below is the final cassette inserted into the *Rosa26* locus of the mouse genome. It consists of the DsRed2 coding sequence, the 3' UTR based on the LacZ backbone with binding sites for 35 MILI-bound piRNAs, flanking LoxP sites, SV40 polyA signal, FRT-flanked Keo reporter cassette (with its own polyA signal). A9 ES cells were electroporated and 300 clones picked. After screening by Southern blotting (EcoRV was chosen for the 3' arm and AflII for the 5' arm) for correct targeting of the *Rosa26* locus, only clones scoring positive with both probes were retained for further study. DNA ladders used for Southern probing are 1kb Extension Ladder (Invitrogen; cat. no. 10511-012) or 1kb ladder (NEB), as indicated. The clone (#1F1B) was retained for injection into C57BL/6N host embryos at the 8-cell stage, after which seven 100% agouti males were obtained. Backcrosses with C57BL/6j Rj (Janvier labs) wildtype females were performed to transfer the targeted allele to the C57BL/6 background to obtain heterozygous *Rosa26-pi* mice. Heterozygous males and female siblings were intercrossed to obtain *Rosa26-pi* homozygous animals. The presence of the upstream polyA site from SV40 is supposed to generate a transcript that has the DsRed coding sequence and a 3'UTR with the piRNA complementary sequences (flanked by LoxP sites).

### Mouse Piwi immunoprecipitations, 5'-end labelling of RNAs and Northern analysis

### Mouse Piwi Immunoprecipitation

Mouse Piwi antibodies were incubated with approximately 15 µL protein G-Sepharose beads (GE Healthcare, cat. No. 17-0618-01) for 3 h and followed by washing (10 mM Tris-HCl pH 8.0, 150 mM NaCl, 0.05% NP-40) to remove the unbound antibody. The mouse testes was homogenized in a glass tissue homogenizer by douncing in lysis buffer [50 mM Tris-HCl pH 8.0, 150 mM NaCl, 5 mM MgCl2, 1 mM DTT, 0.5% sodium deoxycholate (Sigma, cat. No. 30968), 1% Triton X-100, protease inhibitor (Roche)] and spun down for 15 min at 4°C. The bead was incubated with cleared testes lysate supernatant for 3 h and washed five times (10 mM Tris-HCl pH8.0, 150 mM NaCl, 0.05% NP-40). The immunoprecipitated complex was further subjected to western blot or small RNA libraries preparation.

### Northern blot analysis

Total RNA was extracted from testes by using TriZol reagent (Life technology, cat. No. 15596026) according to the manufacture's guide. Approximately 10 µg RNA was resolved on 1% formaldehyde denaturing agarose gel and transferred onto positively-charged Hybond-N+ Nylon membrane (GE Healthcare, cat. No. RPN203B) through capillary transfer for 18 h in 20 X SSC solution (3 M NaCl, 300 mM Sodium Citrate). RNA was crosslinked to the membrane by UV-corsslinker (Stratalinker, 120 mJ, 254 nm). The membrane was pre-hybridized with pre-warmed Church buffer (65°C, 0.25 M Sodium Phosphate pH 7.2, 1 mM EDTA, 1% BSA, 7% SDS) by incubating for 1 h in a rotating hybridization oven. Denatured radiolabeled probe (95°C, 1 min, cool down on ice) was added into the hybridization bottle filled with new pre-warmed Church buffer, and incubate overnight at 65°C in the hybridization oven. The membrane was washed twice with low-stringency wash buffer (2 x SSC containing 0.1% SDS, 10 min for each wash), and further washed once with high-stringency wash buffer (0.1 x SSC containing 0.1% SDS) for 5 min. The membrane was exposed to Phosphor Storage screens (GE Health) and scanned (Typhoon scanner; GE Health). LINE1 DNA fragment (515 bp- 1680 bp) and IAP was selected as probe. DNA probe was labeled using Random Primed DNA labeling kit (Roche, cat. No. 11004760001) with dCTP[α-32P] (Perkin Elmer, cat. No. NEG013H001MC), and was further purified with microspin G-25 column (GE Healthcare, cat. No. 27-5325-01).

### Small RNA libraries and bioinformatics

### Small RNA libraries

RNAs present in endogenous MILI or MIWI2 complexes from testes of new-born pups (P0) were isolated. Briefly, immunoprecipitations were treated with Proteinase K in 300 µL reaction at 42°C for 15 min (10 mM Tris-HCl pH 7.5, 5 mM EDTA, 0.5% SDS). RNAs present in the sample were purified by phenol-chloroform extraction and precipitation with ethanol. Approximately 20% of the sample was radioactively labeled at the 5' end and mixed with the cold RNA. This mixture of radiolabeled and cold RNA was resolved by 15% urea-PAGE, and the RNAs present in the region (∼24-30 nt) was excised and placed in nuclease-free tube. The gel was crushed in the tube and 400 µl of 0.4 M NaCl was added to it. The RNA was eluted from the gel overnight at 25°C in a thermomixer. Gel pieces were removed by filtering the RNA containing gel with SpinX column (Costar, cat. No. 8160). RNA was directly precipitated with glycogen (10-20 µg) and absolute ethanol (1.0 ml) at -20°C overnight. Next day, RNA samples were centrifuged at 16000 x g for 30 minutes. Pellet was washed once with 75% ethanol and finally dissolved in 6 µl RNase free water. RNA was directly used for library preparation. When total small RNA libraries were required, total RNA from testes of new-born pups (P0) was isolated with TRI-reagent (Life Tech, AM9738). RNAs were resolved by 15% urea-PAGE, and RNAs present in the region (∼15-40 nt) was gel-eluted as mentioned above. After purification they were directly used for library preparation. Libraries were prepared (barcoded at 3' end) using NEBNext® Multiplex Small RNA Library Prep Set for Illumina® (NEB Catalogue No. E7300) following manufacture instructions. Multiple libraries with different barcodes (at 3' end) were mixed in equimolar ratio and sequenced with the Illumina HiSeq 2000 platform (EMBL GeneCore facility) for 50 cycles.

### Small RNA bioinformatics of Rosa26-pi reporter knock-in mouse

Reads were sorted into individual libraries based on the barcodes and the 3' adapter sequences were clipped from the reads using cutadapt (DOI:http://dx.doi.org/10.14806/ej.17.1.200). Reads of at least 15 nucleotides were then aligned to the DsRed2-LoxP-piRNAsites-in-LacZ-LoxP-SV40pA reporter sequence using bowtie (Langmead et al., 2009) allowing no mismatches. Only the reads of 25nt-31nt, which mapped to unique reporter site, were used for subsequent analysis.

The 5' ends of piRNAs were mapped and plotted along the reporter (for each nucleotide we plotted the number of piRNAs starting at that nucleotide normalized to millions of library reads (rpm) if their abundance exceeded 1 rpm) in logarithmic scale. Separate panels show the piRNAs targeting the reporter and those produced from the reporter (Figure 6A). To follow the pattern of piRNA production in respect to the target sites, we aggregated the counts of produced piRNAs from all 35 MILI targeted sites and their downstream sequences and plotted the overall number of piRNAs (rpm) having 5' or 3' end at specific distance from the 5' end of targeting piRNA (Figure 6B).

Produced piRNAs with 5' end distance equal to 0 starts immediately downstream from 5' end of targeting piRNA, the piRNAs with distance -10 share 10 nucleotide 5' end overlap with the targeting piRNA. The y-axis showing the piRNA abundance was scaled to show both low and highly abundant piRNAs. The distribution of the ends of produced piRNAs demonstrated that besides the dominant secondary piRNAs with 5' end created by MILI slicing also additional piRNAs are produced downstream of the 3' end of secondary piRNAs. These additional piRNAs which the inventors call "inchworm" piRNAs associate with both MILI and MIWI2.

To better characterize the distance between 3' end of secondary and 5' end of inchworm piRNAs the inventors plotted the fraction of produced piRNA pairs having specific 3' to 5' end distance Δ. The score for specific distance Δ was calculated as: *score*(*Δ*)*=∑min(M*(*i*)*,N*(*i+Δ*)) where *M*(*i*) is the count of produced secondary piRNAas (in rpm) with 3' end on the plus strand at a particular position *i* and *N*(*i*+Δ) is the count of piRNAs on the same strand which have their 5' end position at *i* + Δ. Similarly, we calculated the scores for the distances of the 5' ends and for 3' to 5' distances of piRNAs produced upstream from the first cleavage site. To visualize which individual piRNA sequences are produced we plotted the sequence level detail of the region encompassing 2nd and 3rd site of the reporter. piRNAs produced in an abundance of at least 1 rpm were shown. To investigate the nucleotide composition of produced piRNAs, we aligned their 5' and 3' ends and calculated the proportion of individual nucleotides in the first and last 10 nucleotides. Strong preference for U was observed for the starting nucleotide of MIWI2 secondary piRNAs and both MILI and MIWI2 inchworm piRNAs. Nucleotide frequency was plotted also for the reporter (DNA) nucleotide immediately downstream of the 3' piRNA end. To visualize the individual piRNA production along the reporter, we plotted the individual piRNAs produced from the reporter as well as reporter targeting piRNAs in color based on the presence of 1U and 10A nucleotide which are hallmarks of primary and secondary piRNAs, respectively.
Aravin, A.A., Sachidanandam, R., Bourc'his, D., Schaefer, C., Pezic, D., Toth, K.F., Bestor, T., and Hannon, G.J. (2008). A piRNA pathway primed by individual transposons is linked to de novo DNA methylation in mice. Molecular cell 31, 785-799.
Bieniossek, C., Imasaki, T., Takagi, Y., and Berger, I. (2012). MultiBac: expanding the research toolbox for multiprotein complexes. Trends in biochemical sciences 37, 49-57.
Brennecke, J., Aravin, A.A., Stark, A., Dus, M., Kellis, M., Sachidanandam, R., and Hannon, G.J. (2007).
Discrete small RNA-generating loci as master regulators of transposon activity in Drosophila. Cell 128, 1089-1103.
Cora, E., Pandey, R.R., Xiol, J., Taylor, J., Sachidanandam, R., McCarthy, A.A., and Pillai, R.S. (2014). The MID-PIWI module of Piwi proteins specifies nucleotide- and strand-biases of piRNAs. Rna 20, 773-781.
De Fazio, S., Bartonicek, N., Di Giacomo, M., Abreu-Goodger, C., Sankar, A., Funaya, C., Antony, C., Moreira, P.N., Enright, A.J., and O'Carroll, D. (2011). The endonuclease activity of Mili fuels piRNA amplification that silences LINE1 elements. Nature 480, 259-263.
Di Giacomo, M., Comazzetto, S., Saini, H., De Fazio, S., Carrieri, C., Morgan, M., Vasiliauskaite, L., Benes, V., Enright, A.J., and O'Carroll, D. (2013). Multiple epigenetic mechanisms and the piRNA pathway enforce LINE1 silencing during adult spermatogenesis. Molecular cell 50, 601-608.
Fromm, S.A., Truffault, V., Kamenz, J., Braun, J.E., Hoffmann, N.A., Izaurralde, E., and Sprangers, R. (2012). The structural basis of Edc3- and Scd6-mediated activation of the Dcp1:Dcp2 mRNA decapping complex. The EMBO journal 31, 279-290.
Ghildiyal, M., and Zamore, P.D. (2009). Small silencing RNAs: an expanding universe. Nature reviews Genetics 10, 94-108.
Gunawardane, L.S., Saito, K., Nishida, K.M., Miyoshi, K., Kawamura, Y., Nagami, T., Siomi, H., and Siomi, M.C. (2007). A slicer-mediated mechanism for repeat-associated siRNA 5' end formation in Drosophila. Science 315, 1587-1590.
Han, B.W., Wang, W., Li, C., Weng, Z., and Zamore, P.D. (2015). Noncoding RNA. piRNA-guided transposon cleavage initiates Zucchini-dependent, phased piRNA production. Science 348, 817-821.
Handler, D., Olivieri, D., Novatchkova, M., Gruber, F.S., Meixner, K., Mechtler, K., Stark, A., Sachidanandam, R., and Brennecke, J. (2011). A systematic analysis of Drosophila TUDOR domaincontaining proteins identifies Vreteno and the Tdrd12 family as essential primary piRNA pathway factors. The EMBO journal 30, 3977-3993.
Homolka, D., Pandey, R.R., Goriaux, C., Brasset, E., Vaury, C., Sachidanandam, R., Fauvarque, M.O., and Pillai, R.S. (2015). PIWI Slicing and RNA Elements in Precursors Instruct Directional Primary piRNA Biogenesis. Cell reports 12, 418-428.
Houwing, S., Kamminga, L.M., Berezikov, E., Cronembold, D., Girard, A., van den Elst, H., Filippov, D.V., Blaser, H., Raz, E., Moens, C.B., et al. (2007). A role for Piwi and piRNAs in germ cell maintenance and transposon silencing in Zebrafish. Cell 129, 69-82.
Ishizu, H., Iwasaki, Y.W., Hirakata, S., Ozaki, H., Iwasaki, W., Siomi, H., and Siomi, M.C. (2015). Somatic Primary piRNA Biogenesis Driven by cis-Acting RNA Elements and transActing Yb. Cell reports 12, 429-440.
Kawaoka, S., Hayashi, N., Suzuki, Y., Abe, H., Sugano, S., Tomari, Y., Shimada, T., and Katsuma, S. (2009). The Bombyx ovary-derived cell line endogenously expresses PIWI/PIWI-interacting RNA complexes. Rna 15, 1258-1264.
Kuramochi-Miyagawa, S., Watanabe, T., Gotoh, K., Totoki, Y., Toyoda, A., Ikawa, M., Asada, N., Kojima, K., Yamaguchi, Y., Ijiri, T.W., et al. (2008). DNA methylation of retrotransposon genes is regulated by Piwi family members MILI and MIWI2 in murine fetal testes. Genes & development 22, 908-917.
Li, X.Z., Roy, C.K., Dong, X., Bolcun-Filas, E., Wang, J., Han, B.W., Xu, J., Moore, M.J., Schimenti, J.C., Weng, Z., et al. (2013). An ancient transcription factor initiates the burst of piRNA production during early meiosis in mouse testes. Molecular cell 50, 67-81.
Midtgaard, S.F., Assenholt, J., Jonstrup, A.T., Van, L.B., Jensen, T.H., and Brodersen, D.E. (2006). Structure of the nuclear exosome component Rrp6p reveals an interplay between the active site and the HRDC domain. Proceedings of the National Academy of Sciences of the United States of America 103, 11898-11903.
Mohn, F., Handler, D., and Brennecke, J. (2015). Noncoding RNA. piRNA-guided slicing specifies transcripts for Zucchini-dependent, phased piRNA biogenesis. Science 348, 812-817.
Pandey, R.R., Tokuzawa, Y., Yang, Z., Hayashi, E., Ichisaka, T., Kajita, S., Asano, Y., Kunieda, T., Sachidanandam, R., Chuma, S., et al. (2013). Tudor domain containing 12 (TDRD12) is essential for secondary PIWI interacting RNA biogenesis in mice. Proceedings of the National Academy of Sciences of the United States of America 110, 16492-16497.
Reuter, M., Berninger, P., Chuma, S., Shah, H., Hosokawa, M., Funaya, C., Antony, C., Sachidanandam, R., and Pillai, R.S. (2011). Miwi catalysis is required for piRNA amplification-independent LINE1 transposon silencing. Nature 480, 264-267.
Saxe, J.P., Chen, M., Zhao, H., and Lin, H. (2013). Tdrkh is essential for spermatogenesis and participates in primary piRNA biogenesis in the germline. The EMBO journal 32, 1869-1885. Schumperli, D., and Pillai, R.S. (2004). The special Sm core structure of the U7 snRNP: farreaching significance of a small nuclear ribonucleoprotein. Cellular and molecular life sciences : CMLS 61, 2560-2570.
Wasmuth, E.V., Januszyk, K., and Lima, C.D. (2014). Structure of an Rrp6-RNA exosome complex bound to poly(A) RNA. Nature 511, 435-439.
Xiol, J., Spinelli, P., Laussmann, M.A., Homolka, D., Yang, Z., Cora, E., Coute, Y., Conn, S., Kadlec, J., Sachidanandam, R., et al. (2014). RNA clamping by Vasa assembles a piRNA amplifier complex on transposon transcripts. Cell 157, 1698-1711.
Zuo, Y., and Deutscher, M.P. (2001). Exoribonuclease superfamilies: structural analysis and phylogenetic distribution. Nucleic acids research 29, 1017-1026.

## Claims

1. A method for producing at least one artificial piRNA, comprising the steps of:
a) determining an endogenous piRNA population in a cell of an organism of interest; wherein said organism of interest is capable of methylating its DNA and/or histones;
b) selecting a sub-population of said endogenous piRNA population based on binding of a MILI protein and/or an equivalent PIWI protein to said sub-population;
c) identifying the sequence of at least one piRNA as selected in step b),
d) attaching preferably the complement of said at least one piRNA sequence as identified to an antisense nucleic acid sequence to a gene of interest, in order to generate at least one piRNA template, and
e) slicing said piRNA template through binding of a MILI protein to said template and subsequent slicing of said bound piRNA template following a binding of MILI protein or an equivalent PIWI protein that has small RNA-guided endoribonuclease (slicer) activity, such as MILI, in order to generate at least one artificial piRNA; and
f) optionally, isolating said at least one artificial piRNA from said cell of said organism of interest.

2. The method according to claim 1, wherein said cell is selected from an animal or a cell line, such as, for example, a germ line cell, in particular a female cell, a stem cell, such as, for example, an embryonic stem cell, a reprogrammed stem cell, a somatic cell, an ovarian somatic cell, a tissue, and recombinant cells or tissues thereof.

3. The method according to claim 1 or 2, wherein the organism of interest is capable of methylating its DNA and/or histones is selected from an animal, a mammal, such as, for example, a mouse, rat, cat, dog, horse, goat, sheep, monkey, or human, or a plant, such as, for example, a crop plant, and recombinant animals or plants thereof.

4. The method according to any of claims 1 to 3, wherein selecting the sub-population in step b) comprises identifying the population of endogenous piRNAs using a suitable method, such as, for example, deep sequencing, and or immune-precipitation of MILI protein or equivalent PIWI protein bound to piRNA.

5. The method according to any of claims 1 to 4, wherein selecting the sub-population in step b) comprises using a reporter construct comprising between 10 to 50 complementary sites for MILI-bound piRNAs.

6. The method according to any of claims 1 to 5, wherein the proteins as used, for example MILI and MIWI2, are at least in part recombinantly introduced into the cell and expressed.

7. The method according to any of claims 1 to 6, wherein selecting the sub-population is based, at least in part, on the abundancy of the piRNA sequences in said cell.

8. The method according to any of claims 1 to 7, wherein selecting the number of different artificial piRNAs templates or artificial piRNAs as produced is between 1 and 50, and preferably between 5 and 15.

9. The method according to any of claims 1 to 8, wherein the gene of interest is selected from genes involved in cancer, neurological diseases, metabolic diseases, fertility, animal production, environmental stress genes, genes essential for cell growth and/or development, for mRNA degradation, for translational repression, and for transcriptional gene silencing.

10. The method according to any of claims 1 to 9, further comprising identifying the at least one artificial piRNA as produced.

11. A pharmaceutical composition comprising at least one artificial piRNA as produced according to a method according to any of claims 1 to 10, together with pharmaceutically acceptable salts, esters or salts of such esters, or bioequivalent compounds thereof, admixed, encapsulated, conjugated or otherwise associated with liposomes, polymers, receptor targeted molecules, oral, rectal, topical or other formulations that assist uptake, distribution and/or absorption, and optionally further comprising penetration enhancers, carrier compounds, and/or transfection agents.

12. A method for silencing the expression of a gene of interest in an organism of interest that is capable of methylating its DNA and/or histones, comprising administering to said organism an effective amount of the pharmaceutical composition according to claim 11.

13. The method according to claim 12, wherein said organism suffers or is likely to suffer from a condition or disease that can be treated and/or ameliorated by silencing the expression thereof.

14. A method for produing a non-genetically modified organism having an artificially silenced expression of at least one gene of interest, comprising the steps of:
a) determining an endogenous piRNA population in a germ line or stem cell of said organism; wherein said organism is capable of methylating its DNA and/or histones;
b) selecting a sub-population of said endogenous piRNA population based on binding of a MILI protein and/or an equivalent PIWI protein to said sub-population;
c) identifying the sequence of at least one piRNA as selected in step b),
d) attaching preferably the complement of said at least one piRNA sequence as identified 5' to an antisense nucleic acid sequence to said gene of interest, in order to generate at least one piRNA template, and
e) introducing said piRNA template into the germ line or stem cell of said organism, and
f) generating said non-genetically modified organism through breeding and crossing out of said piRNA template.

15. The method according to claim 14, wherein said gene of interest is selected from genes involved in cancer, neurological diseases, metabolic diseases, fertility, animal production, genes essential for cell growth and/or development, for mRNA degradation, for translational repression, and for transcriptional gene silencing.
